(19) **Europäisches Patentamt / European Patent Office / Office européen des brevets**

(11) **EP 4 134 065 A1**

(12) # EUROPEAN PATENT APPLICATION
published in accordance with Art. 153(4) EPC

(43) Date of publication:
**15.02.2023 Bulletin 2023/07**

(21) Application number: **21784064.4**

(22) Date of filing: **29.01.2021**

(51) International Patent Classification (IPC):
*A61K 8/02* (2006.01)      *A61K 8/19* (2006.01)
*A61K 8/26* (2006.01)      *A61K 8/73* (2006.01)
*A61K 8/81* (2006.01)      *A61K 9/70* (1974.07)
*A61Q 19/00* (2006.01)

(52) Cooperative Patent Classification (CPC):
**A61K 8/02; A61K 8/19; A61K 8/26; A61K 8/73;
A61K 8/81; A61K 9/70; A61K 33/00; A61K 47/02;
A61K 47/32; A61K 47/38; A61P 3/00; A61P 9/08;
A61P 17/00; A61Q 19/00**

(86) International application number:
**PCT/JP2021/003233**

(87) International publication number:
**WO 2021/205719 (14.10.2021 Gazette 2021/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.04.2020 JP 2020069396**

(71) Applicant: **Kao Corporation
Chuo-Ku
Tokyo 103-8210 (JP)**

(72) Inventor: **KIMURA, Shohei
Tokyo 131-0044 (JP)**

(74) Representative: **Hoffmann Eitle
Patent- und Rechtsanwälte PartmbB
Arabellastraße 30
81925 München (DE)**

(54) **METHOD FOR PRODUCING CARBON DIOXIDE-CONTAINING HYDROGEL ARTICLE**

(57)    A method for producing a carbon dioxide-containing hydrogel article including steps (1) to (3). (1) Preparing an article including a hydrogel that contains a water-soluble polymer, a cross-linking agent for the water-soluble polymer, and water, wherein a loss tangent of the hydrogel at 1 Hz in dynamic viscoelasticity measurement is 0.46 or greater. (2) While the loss tangent, at 1 Hz in dynamic viscoelasticity measurement, of the hydrogel in the article including the hydrogel is 0.46 or greater, adjusting the composition of an ambient atmosphere of the article including the hydrogel such that a carbon dioxide gas concentration in the ambient atmosphere becomes 10 vol% or greater, and sealing the article including the hydrogel inside a package. (3) Making cross-linking of the hydrogel proceed until the loss tangent, at 1 Hz in dynamic viscoelasticity measurement, of the hydrogel in the article including the hydrogel becomes less than 0.46, and thereby obtaining a carbon dioxide-containing hydrogel article sealed inside the package.

Fig. 1

EP 4 134 065 A1

**Description**

Technical Field

The present invention relates to a method for producing a hydrogel article containing carbon dioxide gas.

Background Art

Conventional techniques related to steps for including soluble gas in a viscous material are disclosed, for example, in Patent Literatures 1 and 2. Patent Literature 1 discloses a technique involving: introducing a viscous material in a pressure tank filled with a soluble gas under high pressure; and causing the soluble gas to dissolve into the viscous material by stirring with a mixer or by using a porous body.

Patent Literature 2 discloses a technique involving: preparing a raw material liquid of a hydrogel by mixing carboxymethyl cellulose and dried aluminum hydroxide gel; aging the hydrogel to facilitate ion cross-linking reaction of the hydrogel to obtain a cross-linked hydrogel sheet; and then sealing the hydrogel sheet inside a package in which the carbon dioxide gas concentration is 90% or higher, to cause the carbon dioxide gas to dissolve into the hydrogel sheet.

Citation List

Patent Literature

Patent Literature 1: JP2014-62087A
Patent Literature 2: US2020/100991A1

Summary of Invention

The present invention provides a method for producing a carbon dioxide-containing hydrogel article, the method involving steps (1) to (3) below:

(1) a step of preparing an article including a hydrogel that contains (A) a water-soluble polymer, (B) a cross-linking agent for the water-soluble polymer, and (C) water, a loss tangent of the hydrogel at 1 Hz in dynamic viscoelasticity measurement being 0.46 or greater;
(2) a step of, while the loss tangent, at 1 Hz in dynamic viscoelasticity measurement, of the hydrogel in the article including the hydrogel is 0.46 or greater,

adjusting composition of an ambient atmosphere of the article including the hydrogel such that a carbon dioxide gas concentration in the ambient atmosphere becomes 10 vol% or greater, and
sealing the article including the hydrogel inside a package; and

(3) a step of making cross-linking of the hydrogel proceed until the loss tangent, at 1 Hz in dynamic viscoelasticity measurement, of the hydrogel in the article including the hydrogel becomes less than 0.46, and thereby obtaining a carbon dioxide-containing hydrogel article sealed inside the package.

Brief Description of Drawings

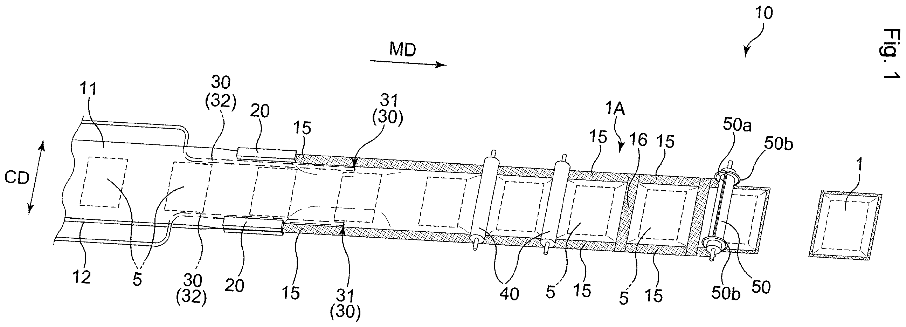

[Fig. 1] Fig. 1 is a schematic diagram illustrating a device suitable for performing a method according to the present invention.

Description of Embodiments

Recent years have seen approaches to apply carbon dioxide gas-dissolved hydrogels to various parts of the human body with the aim of promoting blood circulation or for cosmetic purposes. The techniques disclosed in the aforementioned Patent Literatures 1 and 2 are usable for such applications. Hence, in order to apply carbon dioxide gas to the human body efficiently, it is necessary to raise the concentration of carbon dioxide gas included in the hydrogel. Also, producing such articles on a commercial basis will require a production method with which carbon dioxide-containing hydrogel articles can be produced efficiently. Unfortunately, with the techniques disclosed in Patent Literatures 1 and 2, it is not easy to raise the concentration of carbon dioxide gas to be included in a hydrogel, or, even if it were possible to raise the carbon dioxide gas concentration, it would require considerable time to achieve such high concentrations. Furthermore, there is still room for improvement in terms of efficient production of hydrogel articles containing carbon dioxide gas.

The present invention relates to a method for producing carbon dioxide-containing hydrogel articles that is capable of overcoming the aforementioned drawbacks of conventional art.

The present invention is described below according to preferred embodiments thereof. The present invention relates to a method for producing a carbon dioxide-containing hydrogel article. As disclosed in the following description, with the present invention, a carbon dioxide-containing hydrogel article sealed inside a package is produced. The carbon dioxide-containing hydrogel article includes a hydrogel, and the hydrogel contains carbon dioxide gas.

A carbon dioxide-containing hydrogel article having the aforementioned constitution can be suitably produced according to a method involving the following steps (1) to (3).

(1) A step of preparing an article including a hydrogel.
(2) A step of sealing the article including the hydrogel inside a package together with carbon dioxide gas.
(3) A step of cross-linking the hydrogel.

Each of these steps will be described below.

(1) Step of Preparing Article Including Hydrogel:

In the present step, a hydrogel is prepared. The hydrogel contains (A) a water-soluble polymer, (B) a cross-linking agent for the water-soluble polymer, and (C) water. The water-soluble polymer and the cross-linking agent for the water-soluble polymer will be described in detail further below. The hydrogel is obtained by mixing these components (A) to (C). Mixing can be achieved by using any of various mixers.

Preparation of the hydrogel can be performed at atmospheric temperature or under heating. In the present Description, "atmospheric temperature" refers to ambient temperature, and is a temperature in a state where an environment for performing the present production method is not intentionally heated or cooled, and typically refers to a temperature range from 10°C to 35°C.

Preparation of the hydrogel can be performed at atmospheric pressure, or under increased pressure or reduced pressure compared to atmospheric pressure. It is preferable to employ a pressure that is equal to or above [atmospheric pressure - 0.1 MPa].

In the present Description, "atmospheric pressure" refers to ambient pressure, and is a pressure in a state where an environment for performing the present production method is not intentionally pressurized or decompressed, and typically refers to standard atmospheric pressure (1 atmosphere = 0.1 MPa).

In the present step, from the viewpoint of facilitating the inclusion of carbon dioxide gas into the hydrogel, it is preferable to prepare the hydrogel by mixing the water-soluble polymer, the cross-linking agent, and water at atmospheric pressure. In this way, a multitude of bubbles can be included in the obtained hydrogel. That is, a hydrogel containing bubbles can be obtained easily. By providing the hydrogel with bubbles, a large amount of carbon dioxide gas can be included in the hydrogel in the following step (2). Note that the hydrogel prepared in the present step is in a non-cross-linked state, and more specifically, is a hydrogel whose loss tangent at 1 Hz in dynamic viscoelasticity measurement (referred to hereinafter also as "loss tangent (1 Hz)") is 0.46 or greater. Conditions for measuring the loss tangent will be described in detail further below.

It should be noted that, depending on the type of carbon dioxide-containing hydrogel article to be obtained, there may be cases where it is undesirable to include bubbles in the hydrogel from the viewpoint of making the article less prone to peel from the attached surface or from the viewpoint of providing the article with good conformability to the attached surface. In such cases, the hydrogel may be prepared under reduced pressure.

Regardless of the pressure and temperature at the time of preparing the hydrogel, from the viewpoint of efficient production, the time for mixing the aforementioned components (A) to (C) is preferably from 1 to 60 minutes, more preferably from 5 to 30 minutes. The stirring speed at the time of mixing can be varied as appropriate depending on the amount of the hydrogel to be prepared, and for example, in cases of preparing 1 kg of hydrogel, the stirring speed may be preferably from 10 to 80 rpm (revolutions per minute), more preferably from 20 to 70 rpm, even more preferably from 30 to 65 rpm, from the viewpoint of including many bubbles. By mixing under the aforementioned conditions, a multitude of fine bubbles can be created.

There is no particular limitation to the type of mixer to be used, but from the viewpoint of taking in many bubbles efficiently, it is preferable to use a planetary mixer capable of mixing by employing rotation and revolution, or a wet mixer/kneader.

In the present step, with the aim of improving various properties of the carbon dioxide-containing hydrogel article to be produced, it is possible to mix, together with the aforementioned components (A) to (C), components other than the above. For example, a TRPM8 agonist or an oily agent may be used.

In the present step, from the viewpoint of facilitating the inclusion of carbon dioxide gas into the hydrogel, it is preferable to prepare the hydrogel such that the percentage of water constituting the hydrogel becomes high. In this way, in the following step (3), a large amount of carbon dioxide gas can be easily included in the hydrogel. In the present step, from the viewpoint of facilitating the inclusion of carbon dioxide gas into the hydrogel, the hydrogel may be prepared such that the percentage of water constituting the hydrogel is preferably from 20 to 95 mass%, more preferably from 30 to 92 mass%, even more preferably from 40 to 88 mass%, further more preferably from 70 to 88 mass%.

Step of Forming Article Including Hydrogel:

In the present step, it is preferable to apply the hydrogel obtained according to the aforementioned procedure onto a support, and thereby form a sheet-like article having a hydrogel layer (referred to hereinafter also as "hydrogel article") on one surface of the support. Application of the hydrogel can be achieved using any of various applicators. A hydrogel layer is formed by spreading the hydrogel onto one surface of the support with an applicator.

In cases of spreading the hydrogel with an applicator, it is only necessary, for example, to sandwich the hydrogel between two sheets and spread the hydrogel uniformly. For example, the two sheets for sandwiching the hydrogel may each independently be a nonwoven fabric and a resin film such as a PET film.

For the applicator, from the viewpoint of forming a flat smooth hydrogel layer, it is preferable to use a roll coater, and more preferably a 2-roll coater. By using such an applicator, it is possible to spread the hydrogel by passing the support between the two rolls while applying a strong constant pressure thereto. As a result, the hydrogel layer can be formed in a state where the bubbles in the hydrogel are maintained while keeping the hydrogel supported on the support. Examples of 2-roll coaters may include a direct gravure coater, a chambered doctor coater, a double roller applicator, etc.

In cases of producing a sheet-like hydrogel article in which a hydrogel layer is formed on one surface of a support, from the viewpoint of facilitating the inclusion of carbon dioxide gas into the hydrogel, it is preferable that the hydrogel is applied such that the thickness of the hydrogel layer in the carbon dioxide-containing hydrogel article to be obtained is preferably 0.5 mm or greater, more preferably 0.7 mm or greater, even more preferably 1 mm or greater, whereas, from the viewpoint of making the carbon dioxide-containing hydrogel article less prone to peel from the attached surface and also from the viewpoint of providing the article with good conformability to the attached surface, it is preferable that the hydrogel is applied such that the thickness is preferably 5 mm or less, more preferably 3 mm or less, even more preferably 2 mm or less.

(2) Step of Sealing Hydrogel Article Inside Package:

In the present step, the hydrogel article is sealed inside a package together with carbon dioxide gas in order to make the hydrogel in the hydrogel article absorb carbon dioxide gas. In this case, carbon dioxide gas may be enclosed within the package after placing the hydrogel article inside the package, or the process may be performed in reverse order, or, from the viewpoint of increasing the production amount per day (daily production output), it is preferable to simultaneously perform enclosure of the carbon dioxide gas and sealing of the hydrogel article inside the package.

Since the hydrogel includes both the water-soluble polymer and the cross-linking agent for the water-soluble polymer, cross-linking of the water-soluble polymer starts as a result of these two components coming into contact with one another. Cross-linking of the water-soluble polymer forms a fine mesh structure serving as a fundamental skeleton of the hydrogel. The hydrogel, in which the water-soluble polymer is chemically cross-linked, is in a swollen state as a result of retaining a large amount of water in its mesh structure, and thus has moderate elasticity, extensibility, and flexibility.

If carbon dioxide gas is to be included in the hydrogel after completion of cross-linking or at the terminal stage of cross-linking, time will be necessary for making the hydrogel absorb the carbon dioxide gas. This is not advantageous from the viewpoint of increasing daily production output. So, the present invention is devised to include carbon dioxide gas in the hydrogel at a stage in which the degree of cross-linking of the water-soluble polymer is still low. In this way, carbon dioxide gas can be included in the hydrogel in a short time and with excellent productivity, and thus, daily production output can be increased.

It is not easy to quantitatively evaluate the degree of cross-linking of the water-soluble polymer included in the hydrogel. Inventor conducted various studies from this viewpoint, and has found that it is advantageous to evaluate the degree of cross-linking of the water-soluble polymer by employing the hydrogel's dynamic viscoelastic modulus as a yardstick. Dynamic viscoelastic moduli include storage elastic modulus, loss elastic modulus, and loss tangent. Inventor has found that it is the most objective to evaluate the degree of cross-linking of the water-soluble polymer by employing loss tangent, among the above, as a yardstick.

As described above, in the present invention, the degree of cross-linking of the water-soluble polymer is evaluated based on the loss tangent of the hydrogel. In a state where the degree of cross-linking of the water-soluble polymer is low, the loss tangent value is relatively high; as cross-linking proceeds, the hydrogel's loss tangent value decreases. So, in the present invention, the hydrogel is made to absorb carbon dioxide gas: while the hydrogel's loss tangent, at 1 Hz in dynamic viscoelasticity measurement, is 0.46 or greater, preferably 0.48 or greater, more preferably 0.50 or greater, even more preferably 0.55 or greater, from the viewpoint of increasing daily production output and also from the viewpoint of facilitating the inclusion of carbon dioxide gas into the hydrogel; and while the hydrogel's loss tangent is preferably 0.90 or less, more preferably 0.80 or less, from the viewpoint of increasing daily production output; and while the hydrogel's loss tangent is more preferably 0.65 or less, further more preferably 0.64 or less, from the viewpoint of facilitating the inclusion of carbon dioxide gas into the hydrogel. The method for measuring the loss tangent is described below. In the present invention, a value found at a frequency of 1 Hz is employed as the loss tangent value. This is because this frequency appropriately reflects the cross-linked state of the hydrogel.

In the present invention, the hydrogel's loss tangent (1 Hz) can be measured according to the following method. Measurement is performed at atmospheric temperature and atmospheric pressure, and other measurement conditions are as follows. The loss elastic modulus and storage elastic modulus are found through measurement, and the loss tangent ($\tan\delta$ value) is calculated from these values. In finding the hydrogel's loss tangent, the hydrogel is cut out in a circle having a diameter of 25 mm, and measurement is performed by fixing the hydrogel between parallel plates with a double-faced adhesive tape.

The measurement is performed a plurality of times (at least three times), and the average value is found as the loss tangent ($\tan\delta$ value).

Device: Physica MCR301 rheometer (from Anton Paar)
Sample unit: 25-mm-dia. parallel plates
Normal force: 1 N
Gap distance: 1-2 mm
Mass of hydrogel: 0.5-1.0 g
Frequency: 1 Hz
Strain: 2%
Temperature: 25°C

To make the hydrogel absorb carbon dioxide gas, it is preferable to make the ambient atmosphere of the hydrogel article rich with carbon dioxide gas. From this viewpoint, it is preferable to adjust the composition of the ambient atmosphere of the hydrogel article such that the carbon dioxide gas concentration in the ambient atmosphere becomes preferably 10 vol% or greater while the hydrogel's loss tangent is equal to or greater than the aforementioned value-i.e., while cross-linking of the water-soluble polymer has not yet proceeded excessively. By setting the carbon dioxide gas concentration in the ambient atmosphere to this value or greater, the hydrogel can be made to absorb carbon dioxide gas efficiently. From this viewpoint, it is more preferable to set the carbon dioxide gas concentration in the ambient atmosphere to 30 vol% or greater, even more preferably 60 mass% or greater, further more preferably 80 vol% or greater. The higher the carbon dioxide gas concentration in the ambient atmosphere, the more preferable in terms of efficient absorption of carbon dioxide gas, with the ideal concentration being 100 vol%.

The types of other gas components included in the ambient atmosphere for when making the hydrogel absorb carbon dioxide gas are not particularly limited, so long as the ambient atmosphere includes carbon dioxide gas. Industrially, it is convenient to mix carbon dioxide gas with atmospheric air to raise the concentration of carbon dioxide gas in the ambient atmosphere.

From the viewpoint of efficient absorption of carbon dioxide gas, it is sufficient if the carbon dioxide gas concentration in the ambient atmosphere once becomes 10 vol% or greater while the hydrogel's loss tangent is equal to or greater than the aforementioned value, and the carbon dioxide gas concentration may drop below 10 vol% thereafter. It goes without saying, however, that the carbon dioxide gas concentration in the ambient atmosphere may be kept at 10 vol% or greater while the hydrogel is made to absorb carbon dioxide gas.

The present step can be performed at atmospheric temperature or under heating. Further, the present step can be performed at atmospheric pressure, or under increased pressure or reduced pressure compared to atmospheric pressure. In the present invention, carbon dioxide gas is absorbed by the hydrogel in the initial stage of cross-linking of the water-soluble polymer; hence, the hydrogel absorbs carbon dioxide gas efficiently even without heating. For the same reason, the hydrogel absorbs carbon dioxide gas efficiently even without pressurization. From the viewpoint of increasing daily production output, it is not necessarily advantageous to perform heating and/or pressurization. So, it is preferable to perform the present step at atmospheric temperature and atmospheric pressure. In this way, the intended carbon dioxide-containing hydrogel article can be obtained productively.

Fig. 1 illustrates a structure of an embodiment of a device for performing the present steps. The production device 10 illustrated in the figure includes a first film joining section 20, nozzle sections 30, a second film joining section 40, and a cutter section 50.

The first film joining section 20 is a device for joining, along a transporting direction MD, both lateral sides of original sheets of films transported in the transporting direction MD. As illustrated in Fig. 1, a first original sheet 11 of an air-impermeable film (referred to hereinafter as "first film original sheet 11") being transported in the transporting direction MD has the same width as a second original sheet 12 of an air-impermeable film (referred to hereinafter as "second film original sheet 12") being transported in the same direction as the original sheet 11, and the sheets are supplied to the first film joining section 20 in a state superposed on one another such that the positions of both lateral side edges of the original sheets 11 and 12 match one another.

As illustrated in Fig. 1, the two original sheets 11 and 12 are transported with a transportation means (not illustrated), such as a transportation roller or conveyor, with the hydrogel article 5 arranged between the original sheets, and are supplied to the first film joining section 20. The loss tangent (1 Hz) of the hydrogel in the hydrogel article 5 at this point is equal to or greater than the aforementioned value. As the original sheets 11, and 12 pass through the first film joining section 20 while being transported in a state superposed on one another such that the positions of the original sheets' both lateral side edges match one another, both lateral sides, which are the regions to be joined, of the original sheets 11 and 12 are continuously joined together along the transporting direction MD, thereby forming first joined regions 15 and 15. After the original sheets 11 and 12 pass through the first film joining section 20, the hydrogel article 5 is transported, together with the original sheets, in a state located between the first joined regions 15 and 15-which have been formed respectively on both lateral sides of the original sheets 11 and 12-in a cross direction CD orthogonal to the transporting direction MD (referred to hereinafter as "width direction CD"). For the joining means in the first film joining section 20, it is possible to use, for example, heat sealing, etc. From the viewpoint of joining the films together efficiently, it is preferable that, as illustrated in the figure, the first film joining section 20 is provided as a pair located outside the respective lateral side edges of the hydrogel article 5 in the width direction CD.

The nozzle sections 30 are for supplying carbon dioxide-containing gas to be enclosed within the package. Each nozzle section 30 is a hollow member through which gas can flow; one end of the nozzle section 30 is located upstream of the first film joining section 20 in the transporting direction MD, and an open end 31 constitutes the other end of the nozzle section 30. The nozzle section 30 is configured such that carbon dioxide-containing gas, which is supplied from a gas source (not illustrated) connected to the one end, can be continuously or intermittently supplied toward outside from the open end 31, which is the other end of the nozzle section 30. The open end 31 of the nozzle section 30 is located downstream of the first film joining section 20 in the transporting direction MD and inward of the respective first joined region 15 in the CD direction. The nozzle sections 30 illustrated in Fig. 1 include a pair of nozzle extension portions 32 between the two original sheets 11 and 12, the nozzle extension portions extending along the transporting direction MD. With this configuration, a gas flow is supplied along the transporting direction MD of the original sheets 11 and 12. The nozzle extension portions 32 are arranged substantially parallel to one another. The nozzle extension portions 32 of the nozzle section 30 are located inward of the respective first film joining sections 20 in the width direction CD, and are arranged outward of the respective edges of the hydrogel article 5 being transported. On the upstream side of the first film joining section 20 in the transporting direction, the nozzle sections 30 illustrated in the figure each have a section that is bent inwardly from outside the respective lateral sides of the original sheets 11 and 12. With this configuration, the nozzle extension portions 32 are arranged between the original sheets 11 and 12.

By supplying carbon dioxide-containing gas through the nozzle sections 30, 90 vol% or more of the atmospheric air that is present in the space defined by the two original sheets 11 and 12 can be replaced by carbon dioxide-containing gas. So, if the carbon dioxide-containing gas is carbon dioxide gas itself, then the concentration of gas within the space defined by the two original sheets 11, and 12 can be made substantially 100 vol% carbon dioxide gas.

The second film joining section 40 is arranged on the downstream side, in the transporting direction MD, of the position where the nozzle sections 30 are arranged. The second film joining section 40 is a device configured to join the two original sheets 11 and 12-whose lateral sides have been joined together by being passed through the first film joining section 20-in a direction intersecting with the transporting direction MD. The second film joining section 40 illustrated in the figure takes the configuration of heat rollers. By joining the two original sheets 11 and 12 together at predetermined intervals in a direction intersecting with the transporting direction MD in a state where the hydrogel article 5 is located between the two heat rollers constituting the second film joining section 40, it is thereby possible to form a continuous package 1A in which the original sheets 11 and 12 are joined together at the peripheral edges of the hydrogel article 5. For the joining means in the second film joining section 40, it is possible to use, for example, heat sealing, etc.

The second film joining section 40 is configured to form second joined regions 16 and 16, which extend along the entire region in the width direction CD, by joining together the two original sheets 11 and 12-whose lateral sides have been joined together by being passed through the first film joining section 20-in a direction intersecting with the transporting direction MD. The second film joining section 40 illustrated in Fig. 1 takes the configuration of heat rollers. In a state before forming the second joined regions 16, the second film joining section 40 is arranged such that regions of both original sheets 11 and 12 other than the first joined regions 15 are in a separated state, and preferably, the second film joining section 40 is separated from the original sheets 11 and 12. When the hydrogel article 5 comes to be located between the second film joining section 40 arranged along the transporting direction MD, the second film joining section 40 is controlled, by a control means (not illustrated) for moving the second film joining section 40, so as to be pressed against the original sheets 11 and 12 such that it abuts against the original sheets 11 and 12, to thereby join the original sheets 11 and 12 together in a direction intersecting with the transporting direction MD. In this way, the original sheets 11 and 12, which are located outside the peripheral edges of the hydrogel article 5, are joined together by the pair of first joined regions 15 and 15 and the pair of second joined regions 16, and 16. Thus, it is possible to form a continuous package 1A in which the hydrogel article 5 and the carbon dioxide-containing gas are enclosed within the joined regions 15 and 16 in a sealed state.

The cutter section 50 is arranged on the downstream side, in the transporting direction MD, of the position where the second film joining section 40 is arranged. The cutter section 50 includes a cutter blade extending along a direction intersecting with the transporting direction MD, and can cut the original sheets 11 and 12 at the position of the second joined region 16 in the continuous package 1A, to thereby cut and separate the continuous package into single pieces of packages 1. The cutter section 50 illustrated in Fig. 1 takes the configuration of a cutter roller whose axial direction matches the width direction CD, and includes a first cutter blade 50a provided on the circumference of the roller and extending along the axial direction of the roller, and second cutter blades 50b extending along the circumferential direction of the roller. By the rotation of the cutter roller, the first cutter blade 50a cuts the continuous package 1A at the position of the second joined region 16 along a direction intersecting with the transporting direction MD. The second cutter blades 50b are arranged as a pair on the continuous package 1 A's both end sides in the width direction CD, and can thus cut the continuous package 1A at the positions of the respective first joined regions 15 along the transporting direction MD. In this way, it is possible to form a package 1 in which the carbon dioxide-containing gas and the hydrogel article are sealed.

In the device having the aforementioned configuration, the nozzle sections 30 are located inward of the first film joining section 20 in the width direction CD, and the open end 31 of each nozzle section 30 is located on the downstream side of the first film joining section 20 in the transporting direction. Thus, the nozzle sections 30 serve as guides for regulating the joining positions of the original sheets 11 and 12, and thus, it is possible to join the original sheets 11 and 12 together along the transporting direction at desired positions. Further, at the time of joining the films together, the nozzle sections 30 regulate the movement of the hydrogel article 5, and thus, it is possible to prevent the hydrogel article 5 from getting entangled or caught unintentionally by the first film joining section 20. As a result, the hydrogel article 5 can be prevented from getting damaged, and the package 1 can be produced stably and continuously. In addition, arranging the nozzle sections 30 along the transporting direction MD so as to extend downstream of the first film joining section 20 in the transporting direction is also advantageous in that the atmospheric air inside the original sheets 11, and 12 can be replaced more efficiently by the carbon dioxide-containing gas compared to cases where the nozzle sections 30 are inserted in a direction intersecting with the transporting direction MD.

As described above, with the device having the aforementioned configuration, it is possible to continuously perform: a first joining step of arranging the hydrogel article between an air-impermeable first original sheet and an air-impermeable second original sheet and supplying a carbon dioxide-containing gas between the two original sheets, and in this state, joining both lateral sides of the two original sheets which are being transported in a single direction, the lateral sides extending along the transporting direction; a second joining step of joining the two original sheets together in a direction intersecting with the transporting direction at a position where the hydrogel article is not present, to thereby form a continuous package in which the hydrogel article and the carbon dioxide-containing gas are enclosed; and a cutting step of cutting the continuous package in a direction intersecting with the transporting direction at a region joined by the second joining step, to thereby form the package. As a result, in addition to allowing carbon dioxide gas to be absorbed while the degree of cross-linking of the water-soluble polymer is still low, it is possible to produce the intended carbon dioxide-containing hydrogel article even more productively.

(3) Step of Cross-Linking Hydrogel:

In the aforementioned step (2), the hydrogel article is sealed within the package together with carbon dioxide-containing gas. In this sealed state, the carbon dioxide gas is absorbed by the hydrogel. Absorption of carbon dioxide gas by the hydrogel proceeds spontaneously. Hence, no particular operation, such as heating of the package, is necessary. That is, absorption of carbon dioxide gas by the hydrogel can be performed at atmospheric temperature. Note that, in cases where it is desired to increase the production rate, for example, the package may be heated in the present step, from the viewpoint of promoting the progress of cross-linking of the water-soluble polymer described below.

In cases where there are bubbles in the hydrogel, the carbon dioxide gas dissolves into the hydrogel by action of osmotic pressure (concentration gradient) within the sealed package and also diffuses into the bubbles. That is, the carbon dioxide gas is not absorbed only by the hydrogel itself, but also into the bubbles in cases where the hydrogel includes bubbles. As the carbon dioxide gas is absorbed, the carbon dioxide gas concentration inside the package gradually decreases, but that is not a problem.

As the carbon dioxide gas is absorbed by the hydrogel, cross-linking of the water-soluble polymer included in the hydrogel proceeds. As a result, the loss tangent of the hydrogel decreases. From the viewpoint of facilitating the inclusion of carbon dioxide gas into the hydrogel, cross-linking of the water-soluble polymer is made to proceed until the loss tangent (1 Hz) becomes less than 0.46, preferably less than 0.40, more preferably less than 0.38, even more preferably less than 0.35. In cases where cross-linking is performed at atmospheric temperature, the time required until the loss tangent becomes less than 0.46 is preferably from 24 to 366 hours, more preferably from 24 to 240 hours, from when the hydrogel article is sealed.

From the viewpoint of facilitating the inclusion of carbon dioxide gas into the hydrogel and also from the viewpoint of improving adhesiveness of the hydrogel article, it is preferable to make cross-linking of the water-soluble polymer proceed until the loss tangent (1 Hz) is preferably from 0.20 to 0.45, more preferably from 0.35 to 0.45, even more preferably from 0.40 to 0.45.

Cross-linking of the water-soluble polymer improves the hydrogel's shape retainability. The concentration of carbon dioxide gas included in the hydrogel at the time of completion of cross-linking is typically from 10 to 20000 ppm, although this depends on the type of water-soluble polymer or the water content of the hydrogel.

The concentration of carbon dioxide gas is found as follows. The carbon dioxide-containing hydrogel article is placed in a lidded container containing an alkaline solution and immersed therein for 12 hours, to trap the carbon dioxide gas in the aqueous solution as a carbonate. Then, the solution is made acidic again with an acidic buffer solution, and the carbon dioxide gas concentration of the solution at room temperature (25°C) is measured with a carbon dioxide gas electrode. Then, from the obtained carbon dioxide gas concentration, the mass of the article and the volume of the solution, the content of carbon dioxide gas in the carbon dioxide-containing hydrogel article is calculated, and this value is considered the aforementioned carbon dioxide gas concentration.

Conventionally, when producing this type of article containing carbon dioxide gas, a long time is required for maturing in order to make the hydrogel absorb carbon dioxide gas. Hence, there is a need to secure space for temporarily storing the articles to make them absorb carbon dioxide gas. In contrast, with the present production method, the conventional maturing is performed at the final stage of the production process, and hence, there is no need to secure space for temporarily storing the articles. More specifically, the articles can be packed in shipping boxes, such as cardboard boxes, immediately after completion of the aforementioned step (3), which is advantageous in that the articles do not need to occupy unnecessary space.

The above process yields carbon dioxide-containing hydrogel articles in which the hydrogel has sufficiently absorbed carbon dioxide gas. The article is preferably used as a body-adhering article which is used by attaching the hydrogel to the body. By attaching the carbon dioxide-containing hydrogel article to the body, the carbon dioxide gas included in the hydrogel is released and acts on the skin, and can thereby promote the user's blood circulation. Regions for adhesion may preferably include, for example, parts of the body other than the scalp, such as the feet, arms, shoulders, hips/lower back, etc. The time for adhesion/application is not particularly limited, but may be preferably 15 minutes or more, more preferably 30 minutes or more. For example, the article may be used as a body-adhering article which the user attaches before going to bed and keeps on during sleep by employing the adhesive force of the hydrogel. Note that the carbon dioxide-containing hydrogel article of the present invention does not necessarily have to be a sheet-like hydrogel article in which the hydrogel is supported by a support such as a nonwoven fabric, but instead, the hydrogel alone may be applied directly to the body.

The type of water-soluble polymer to be used in the aforementioned production method is not particularly limited so long as it is a substance that can retain water stably, and for example, is preferably an anionic polymer from the viewpoint of shapeability.

Examples of the anionic polymer may include polymers having a carboxyl group, sulfuric acid group, or phosphoric acid group. Concrete examples may include: poly(meth)acrylic acid and salts thereof; carboxyvinyl polymer and salts thereof; anionic cellulose derivatives, such as carboxymethyl cellulose, carboxyethyl cellulose, etc., and salts thereof; carrageenan, alginic acid, and salts thereof; and anionic starch derivatives. Among the above, from the viewpoint of obtaining a hydrogel having a particularly large water-retention amount, sufficient gel strength, and flexibility that allows conformation to projections/depressions and movement, it is preferable to use at least one type selected from carboxymethyl cellulose and salts thereof, and poly(meth)acrylic acid and salts thereof, and more preferably sodium carboxymethyl cellulose.

The cross-linking agent for the water-soluble polymer is selected as appropriate depending on the type of water-soluble polymer. For example, in cases where the water-soluble polymer is the aforementioned anionic polymer, it is possible to use, for example, a metal ion compound, a cationic polymer, a polyfunctional epoxy compound, etc., as the cross-linking agent.

Examples of the metal ion compound may include oxides, hydroxides, salts, etc., including aluminum, magnesium, calcium, potassium, etc. For example, it is possible to use at least one type selected from aluminum hydroxide, potassium alum, aluminum sulfate, aluminum oxide, aluminum glycinate, aluminum acetate, aluminum lactate, aluminum stearate, hydrated aluminum silicate, aluminum metasilicate, magnesium aluminometasilicate, magnesium chloride, magnesium stearate, calcium carbonate, calcium hydroxide, kaoline, synthetic hydrotalcite, potassium hydroxide, etc.

In cases of using an anionic polymer that is cross-linkable by action of aluminum, such as carboxymethyl cellulose or a salt thereof, as the water-soluble polymer, it is preferable to use an aluminum ion-supplying cross-linking agent-i.e., an agent capable of supplying aluminum to the water-soluble polymer-as the cross-linking agent, because such an agent has excellent dispersibility and high solubility in the hydrogel. For such a cross-linking agent, it is preferable to use, for example, an aluminum ion compound, such as magnesium aluminometasilicate, aluminum hydroxide, etc. Particularly, magnesium aluminometasilicate not only has excellent dispersibility and high solubility in the hydrogel, but also facilitates cross-linking reaction even at atmospheric temperature, and can thus impart sufficient elasticity to the hydrogel.

In cases of using magnesium aluminometasilicate as the cross-linking agent, it may be used alone, or in combination with other cross-linking agents. In cases of using magnesium aluminometasilicate in combination with other cross-linking agents, from the viewpoint of allowing the water-soluble polymer to be cross-linked sufficiently, it is preferable that the percentage of magnesium aluminometasilicate within the cross-linking agent(s) is preferably from 30 to 100 mass%, more preferably from 70 to 100 mass%.

From the viewpoint of increasing daily production output, it is preferable that, in the step (1), the cross-linking agent is included such that the content of the cross-linking agent in the hydrogel is from 0.01 to 10 mass%. From the viewpoint of further increasing daily production output, it is preferable that the charging amount of the cross-linking agent in the hydrogel is more preferably 0.03 mass% or greater, even more preferably 0.05 mass% or greater, further more preferably 0.1 mass% or greater, whereas from the viewpoint of facilitating the inclusion of carbon dioxide gas into the hydrogel, the content is more preferably 5 mass% or less, even more preferably 2 mass% or less.

From the viewpoint of facilitating the inclusion of carbon dioxide gas into the hydrogel, it is preferable that the carbon dioxide-containing hydrogel article obtained by the present production method includes, in the hydrogel, carbon dioxide gas-containing bubbles at a percentage of preferably 10% or greater, more preferably 20% or greater, in terms of bubble ratio.

On the other hand, from the viewpoint of making the article less prone to peel from the attached surface and from the viewpoint of providing the article with good conformability to the attached surface, it is preferable that the carbon dioxide-containing hydrogel article obtained by the present production method includes, in the hydrogel, carbon dioxide gas-containing bubbles at a percentage of preferably 40% or less, more preferably 35% or less, in terms of bubble ratio. Herein, "bubble ratio" refers to a value measured and calculated according to the following method.

The carbon dioxide-containing hydrogel article obtained by the present production method is sealed inside a sparingly gas-permeable container. So, the bubble ratio is found as a value measured within 5 minutes from when the container is opened and the sheet is taken out. More specifically, assuming that the specific gravity of the portion excluding the bubbles is 1, the sheet taken out from the container is cut into a rectangular parallelepiped shape and the volume A (mL) thereof is found from its length $\times$ width $\times$ height, and also the mass B (g) is measured. Then, the bubble ratio is calculated by substituting these values into equation (1) below.

$$\text{Equation (1): Bubble ratio (\%)} = (A-B)/A \times 100.$$

In cases where the carbon dioxide-containing hydrogel article is supported on a support layer and/or a peeling layer, from the viewpoint of the sake of convenience of operation, measurement may be performed without removing/peeling these layers, and the bubble ratio may be calculated by subtracting the total mass of the support layer and the peeling layer afterward.

In the carbon dioxide-containing hydrogel article obtained by the present production method, it is preferable that the total area of bubbles having virtual diameters of 5 mm or greater within the hydrogel's surface area (assumed as 100%) is 10% or less. By controlling the size of the bubbles, the carbon dioxide gas in the article can be supplied to the skin efficiently. Also, by suppressing coexistence of unnecessarily oversized bubbles, the smoothness of the hydrogel's surface can be improved. Thus, the article's adhesiveness can be enhanced, and the article's attachability to the body can be improved effectively.

In the carbon dioxide-containing hydrogel article obtained by the present production method, the bubbles that are present on the hydrogel's surface are measured at room temperature (25°C) at a relative humidity of 50% within 5 minutes from when the later-described sparingly gas-permeable container is opened and the sealed content, i.e., the carbon dioxide-containing hydrogel article, is taken out therefrom. The bubbles may be amorphous or elliptic; such a bubble is assumed to be a virtual circle whose diameter is found by adding the major axis and minor axis and dividing the sum by two, and the diameter of the virtual circle is defined as the "virtual diameter of the bubble". Hence, "the total area of bubbles having virtual diameters of 5 mm or greater" refers to the sum total found by selecting all bubbles that are present on the article's surface to contact the skin and whose virtual diameter, as defined above, is 5 mm or greater, and adding up the respective virtual circle's area found based on the virtual diameter thereof. The "total area (%) of bubbles having virtual diameters of 5 mm or greater within the hydrogel's surface area (assumed as 100%)" is considered to be a value found by dividing the aforementioned total area by the hydrogel's surface area, assuming that the hydrogel's surface area is 100%.

In cases where such fine bubbles are present over the entire hydrogel layer of the carbon dioxide-containing hydrogel article obtained by the present production method, the bubbles are present not only on the hydrogel's surface, but also inside the hydrogel. So, also for bubbles that are present on a cross section of the hydrogel, the "total area (%) of bubbles having virtual diameters of 5 mm or greater within the cross-sectional area (assumed as 100%)" has the same value as the "total area (%) of bubbles having virtual diameters of 5 mm or greater within the hydrogel's surface area (assumed as 100%)".

From the viewpoint of making the carbon dioxide-containing hydrogel article obtained by the present production method less prone to peel from the attached surface and from the viewpoint of providing the article with good conformability to the attached surface, as described above, the total area of bubbles having virtual diameters of 5 mm or greater within the hydrogel's surface area (assumed as 100%) is preferably 10% or less, more preferably 5% or less, and it is even more preferable that the hydrogel includes no bubbles having virtual diameters of 5 mm or greater within the hydrogel's surface area (assumed as 100%).

Examples of components that may be optionally used at the time of preparing the hydrogel include components typically used in cosmetics and pharmaceutical products, such as oily components, water-soluble polymers, alcohols, sugars, blood circulation promoting agents, skin nutrients, vitamins, antioxidants, antioxidant aids, antiseptics, coloring agents, perfumes, etc.

For the support on which the hydrogel is to be applied, it is possible to use, for example, a sheet-like base material, such as a woven fabric, a nonwoven fabric, a knitted fabric, a synthetic resin film, waterproof paper, etc. It is also possible to use a laminate in which a plurality of such materials are layered. More specifically, usable examples may include: woven fabrics and nonwoven fabrics made from synthetic fibers, such as polyamide, polypropylene, polyester, polyethylene, polystyrene, polyurethane, polyolefin, etc., and/or natural fibers, such as silk, cotton, hemp, rayon, collagen, etc.; sheets made from polyamide, polypropylene, polyester, polyethylene, polyurethane, etc.; and thin film sheets made from pullulan, starch, etc. Among the above, from the viewpoint of reducing the size of the bubbles and from the viewpoint of ensuring good attachability to the body, it is preferable to use a woven fabric or a nonwoven fabric, more preferably a nonwoven fabric.

The thickness of the support is approximately from 0.05 to 2.0 mm. The surface of the support onto which the hydrogel is to be applied may be subjected to a hydrophilizing treatment or hydrophobizing treatment.

Usable examples of air-impermeable materials constituting the package may include: films onto which a metal, such as aluminum etc., has been vapor-deposited; and laminated films made by layering two or more layers of films made from different materials. In cases of employing devices other than the device illustrated in Fig. 1, it is also possible to use glass containers or plastic containers.

The package is air-impermeable, and particularly, needs to be sparingly permeable to carbon dioxide. "Sparingly permeable to carbon dioxide" means that the permeability of carbon dioxide is equal to or less than 50 $cc/(m^2 \cdot day \cdot atm)$ (ASTM D-1434). It is preferable that the package is carbon dioxide-impermeable. Examples of preferable materials for the package may include heat-sealable materials, with concrete examples including: laminated films including a layer of aluminum foil; laminated films including an aluminum vapor deposition layer; polyvinylidene chloride films; and laminated films including a polyvinylidene chloride layer. The shape of the package may preferably be a flat bag or a gusseted bag. If necessary, with the aim of retaining a composition made only from the hydrogel, it is possible to employ a method of using a tray made, for example, from polyethylene terephthalate and filling the tray with the hydrogel, and then storing the same in a package, such as the aforementioned bag.

From the viewpoint of suppressing bulging etc. of the package caused by changes in air temperature etc., it is preferable that the package maintains a volume capable of retaining a filling ratio of 0.5% to 35% with respect to the volumetric capacity.

The present invention has been described above according to preferred embodiments thereof, but the present invention is not limited to the foregoing embodiments. For example, the aforementioned steps (1) to (3) are preferably performed at atmospheric temperature, and more preferably at atmospheric temperature and atmospheric pressure, but, if necessary, one or more of the steps (1) to (3) may be performed under such conditions as cooling, heating, reduced pressure, or increased pressure. Note, however, that from the viewpoint of obtaining the target product efficiently and productively, it is preferable to perform all of the steps (1) to (3) at atmospheric temperature and atmospheric pressure.

In relation to the foregoing embodiments, the present invention further discloses the following methods for producing a carbon dioxide-containing hydrogel article.

{1} A method for producing a carbon dioxide-containing hydrogel article, the method comprising steps (1) to (3) below:

(1) a step of preparing an article including a hydrogel that contains (A) a water-soluble polymer, (B) a cross-linking agent for the water-soluble polymer, and (C) water, a loss tangent of the hydrogel at 1 Hz in dynamic viscoelasticity measurement being 0.46 or greater;

(2) a step of, while the loss tangent, at 1 Hz in dynamic viscoelasticity measurement, of the hydrogel in the article including the hydrogel is 0.46 or greater,

adjusting composition of an ambient atmosphere of the article including the hydrogel such that a carbon dioxide gas concentration in the ambient atmosphere becomes 10 vol% or greater, and
sealing the article including the hydrogel inside a package; and

(3) a step of making cross-linking of the hydrogel proceed until the loss tangent, at 1 Hz in dynamic viscoelasticity measurement, of the hydrogel in the article including the hydrogel becomes less than 0.46, and thereby obtaining a carbon dioxide-containing hydrogel article sealed inside the package.

{2} The method for producing a carbon dioxide-containing hydrogel article as set forth in clause {1}, wherein the step (2) is performed at atmospheric temperature and atmospheric pressure.
{3} The method for producing a carbon dioxide-containing hydrogel article as set forth in clause {1} or {2}, wherein:

the water-soluble polymer contains an anionic polymer; and
the cross-linking agent contains an aluminum ion-supplying cross-linking agent.

{4} The method for producing a carbon dioxide-containing hydrogel article as set forth in any one of clauses {1} to {3}, wherein the step (2) comprises:

a first joining step of arranging the article including the hydrogel between an air-impermeable first original sheet and an air-impermeable second original sheet and supplying a carbon dioxide-containing gas between the two original sheets, and in this state, joining both lateral sides of the two original sheets which are being transported in a single direction, the lateral sides extending along the transporting direction;
a second joining step of joining the two original sheets together in a direction intersecting with the transporting direction at a position where the hydrogel article is not present, to thereby form a continuous package in which the article including the hydrogel and the carbon dioxide-containing gas are enclosed; and
a cutting step of cutting the continuous package in a direction intersecting with the transporting direction at a region joined by the second joining step, to thereby form the package.

{5} The method for producing a carbon dioxide-containing hydrogel article as set forth in any one of clauses {1} to {4}, wherein, in the step (2), the composition of the ambient atmosphere is adjusted such that the carbon dioxide gas concentration in the ambient atmosphere of the article including the hydrogel becomes 10 vol% or greater while the loss tangent is preferably 0.48 or greater, more preferably 0.50 or greater, even more preferably 0.55 or greater, and preferably 0.90 or less, more preferably 0.80 or less, even more preferably 0.65 or less, even more preferably 0.64 or less.
{6} The method for producing a carbon dioxide-containing hydrogel article as set forth in any one of clauses {1} to {5}, wherein the step (1) comprises a step of mixing the water-soluble polymer, the cross-linking agent, and the water at atmospheric pressure, to thereby prepare the hydrogel including bubbles.
{7} The method for producing a carbon dioxide-containing hydrogel article as set forth in any one of clauses {1} to {6}, wherein, in the step (2), while the loss tangent is 0.46 or greater, the composition of the ambient atmosphere is adjusted such that the carbon dioxide gas concentration in the ambient atmosphere of the article including the hydrogel becomes 10 vol% or greater, preferably 30 vol% or greater, more preferably 60 mass% or greater, even more preferably 80 vol% or greater.
{8} The method for producing a carbon dioxide-containing hydrogel article as set forth in any one of clauses {1} to {7}, wherein, in the step (2), an operation of housing the article including the hydrogel inside the package and an operation of supplying a carbon dioxide-containing gas into the package are performed in this order, or performed in reverse order, or performed simultaneously, and then the package is sealed.
{9} The method for producing a carbon dioxide-containing hydrogel article as set forth in any one of clauses {1} to {8}, wherein, in the step (3), the carbon dioxide-containing hydrogel article sealed inside the package is obtained by making cross-linking of the hydrogel proceed until the loss tangent becomes preferably less than 0.40, more preferably less than 0.38, even more preferably less than 0.35.
{10} The method for producing a carbon dioxide-containing hydrogel article as set forth in any one of clauses {1} to {9}, wherein:

the step (3) is performed at atmospheric temperature; and

in the step (3), the time required until the loss tangent becomes less than 0.46 is preferably from 24 to 366 hours, more preferably from 24 to 240 hours, from when the hydrogel article is sealed.

{11} The method for producing a carbon dioxide-containing hydrogel article as set forth in any one of clauses {1} to {10}, wherein, in the step (3), it is preferable to make cross-linking of the hydrogel proceed until the loss tangent is preferably from 0.20 to 0.45, more preferably from 0.35 to 0.45, even more preferably from 0.40 to 0.45.

{12} The method for producing a carbon dioxide-containing hydrogel article as set forth in any one of clauses {1} to {11}, wherein the step (1) comprises a step of applying the prepared hydrogel onto a support, and forming, on the support, the article including the hydrogel.

{13} The method for producing a carbon dioxide-containing hydrogel article as set forth in clause {12}, wherein, in the step of forming, on the support, the article including the hydrogel, the hydrogel is applied such that the thickness of the hydrogel in the carbon dioxide-containing hydrogel article is preferably from 0.5 to 5 mm, more preferably from 0.7 to 3 mm, even more preferably from 1 to 2 mm.

{14} The method for producing a carbon dioxide-containing hydrogel article as set forth in any one of clauses {1} to {13}, wherein, in the step (1), the time for mixing the water-soluble polymer, the cross-linking agent, and the water is preferably from 1 to 60 minutes, more preferably from 5 to 30 minutes.

{15} The method for producing a carbon dioxide-containing hydrogel article as set forth in any one of clauses {1} to {14}, wherein, in the step (1), the stirring speed at the time of mixing the water-soluble polymer, the cross-linking agent, and the water is preferably from 10 to 80 rpm, more preferably from 20 to 70 rpm, even more preferably from 30 to 65 rpm.

{16} The method for producing a carbon dioxide-containing hydrogel article as set forth in any one of clauses {1} to {15}, wherein the water-soluble polymer is an anionic polymer.

{17} The method for producing a carbon dioxide-containing hydrogel article as set forth in clause {16}, wherein the anionic polymer includes a carboxyl group, a sulfuric acid group, or a phosphoric acid group.

{18} The method for producing a carbon dioxide-containing hydrogel article as set forth in clause {17}, wherein the anionic polymer is at least one type selected from: poly(meth)acrylic acid and salts thereof; carboxyvinyl polymer and salts thereof; anionic cellulose derivatives, such as carboxymethyl cellulose, carboxyethyl cellulose, etc., and salts thereof; carrageenan, alginic acid, and salts thereof; and anionic starch derivatives.

{19} The method for producing a carbon dioxide-containing hydrogel article as set forth in any one of clauses {1} to {18}, wherein the cross-linking agent is a metal ion compound, a cationic polymer, or a polyfunctional epoxy compound.

{20} The method for producing a carbon dioxide-containing hydrogel article as set forth in clause {19}, wherein:

the cross-linking agent is a metal ion compound; and

the metal ion compound is an oxide, a hydroxide, or a salt including aluminum, magnesium, calcium, potassium, etc.

{21} The method for producing a carbon dioxide-containing hydrogel article as set forth in clause {20}, wherein the metal ion compound is at least one type selected from aluminum hydroxide, potassium alum, aluminum sulfate, aluminum oxide, aluminum glycinate, aluminum acetate, aluminum lactate, aluminum stearate, hydrated aluminum silicate, aluminum metasilicate, magnesium aluminometasilicate, magnesium chloride, magnesium stearate, calcium carbonate, calcium hydroxide, kaoline, synthetic hydrotalcite, potassium hydroxide, etc.

{22} The method for producing a carbon dioxide-containing hydrogel article as set forth in clause {21}, wherein the metal ion compound is magnesium aluminometasilicate.

{23} The method for producing a carbon dioxide-containing hydrogel article as set forth in clause {22}, wherein the percentage of magnesium aluminometasilicate within the cross-linking agent(s) is preferably from 30 to 100 mass%, more preferably from 70 to 100 mass%.

{24} The method for producing a carbon dioxide-containing hydrogel article as set forth in any one of clauses {1} to {23}, wherein the charging amount of the cross-linking agent in the hydrogel is preferably 0.01 mass% or greater, more preferably 0.03 mass% or greater, even more preferably 0.05 mass% or greater, even more preferably 0.1 mass% or greater, and preferably 10 mass% or less, more preferably 5 mass% or less, even more preferably 2 mass% or less.

{25} The method for producing a carbon dioxide-containing hydrogel article as set forth in any one of clauses {1} to {24}, wherein the charging amount of the water in the hydrogel is preferably from 20 to 95 mass%, more preferably from 30 to 92 mass%, even more preferably from 40 to 88 mass%, further more preferably from 70 to 88 mass%.

Examples

The present invention will be described in further detail below according to Examples. Note, however, that the scope of the present invention is not limited to the following Examples.

Example 1:

Step (1):

The components shown in Table 1 were used in amounts shown in Table 1. At atmospheric pressure and at 25°C, an aqueous solution including 5 g of succinic acid and 50.0 g of polyvinyl alcohol dissolved in 764 g of water was placed in a wet mixer/kneader, and further, 28.0 g of sodium carboxymethyl cellulose, 0.5 g of magnesium aluminometasilicate, 100.0 g of glycerin, 50.0 g of propylene glycol, and 2.5 g of methyl parahydroxybenzoate were added, and the mixture was mixed at a stirring speed of 60 rpm for 40 minutes, to prepare a hydrogel including bubbles.

Step of Forming Article Including Hydrogel:

The hydrogel obtained in Step (1) was sandwiched between a polypropylene film and a nonwoven fabric at atmospheric pressure and at 25°C, and was spread by using a 2-roll coater, to form a hydrogel layer having a thickness of 2 mm. Then, the sheet was die-cut into a rectangular shape having a size of $12.5 \times 8.5$ cm (area: $106.25$ cm$^2$; hydrogel application amount: $1250$ g/m$^2$). In this way, a hydrogel article was obtained, with the hydrogel layer including bubbles being formed on a support made of nonwoven fabric.

Step (2):

The present step was performed using the device illustrated in Fig. 1. When the loss tangent (1 Hz) of the hydrogel in the hydrogel article obtained in the aforementioned step was 0.50 (i.e., after 4 hours from the completion of Step (1)), two sheets of the die-cut hydrogel articles were arranged between two sheets of rectangular aluminum vapor deposition films, and, while supplying carbon dioxide gas between the two films, the four sides of the vapor deposition films were joined together in a hermetically sealed manner, to seal the articles inside a package made of the vapor deposition films. The inner surface area of the package made of the aluminum vapor deposition films was 300 cm$^2$, and the volumetric capacity inside the package was 354 cm$^3$. This step was performed at 25°C and at atmospheric pressure. The method for calculating the carbon dioxide gas concentration in the ambient atmosphere when sealing the package is as described below.

Method for Calculating Carbon Dioxide Gas Concentration in Ambient Atmosphere When Sealing Package: With respect to a hydrogel article packaged inside a sparingly gas-permeable container filled with carbon dioxide gas, the oxygen concentration inside the container is measured, and the carbon dioxide gas concentration inside the container is calculated from the obtained value. More specifically, the oxygen concentration (F%) inside the container is measured with a Zirconia Oxygen Analyzer LS-450F (from Toray Engineering Co., Ltd.). The air has an oxygen concentration of 20.6%; hence, the percentage of air (G%) inside the container can be found by the formula (F/20.6) $\times$ 100. Assuming that gas other than air is carbon dioxide gas, the carbon dioxide gas concentration (H%) inside the container is defined by the formula 100-G.

Method for Calculating Volumetric Capacity Inside Package as Calculated from Inner Surface Area of Package: In the present Description, "volumetric capacity calculated from the inner surface area of the package" is defined as the volumetric capacity found from the volume of a cube that can be assumed from the inner surface area. That is, when each side of a cube is defined as X cm, the surface area S of the cube is X squared times 6 (i.e., $6X^2$) and the volume of the cube is X cubed (i.e., $X^3$). So, assuming that the volume is equal to volumetric capacity, the volumetric capacity can be expressed as $(S/6)^{3/2}$.

Step (3):

By placing the hydrogel article sealed in the package in an environment at 25°C for 120 hours, cross-linking of the hydrogel was made to proceed until the hydrogel's loss tangent was 0.30. In this way, an intended carbon dioxide-containing hydrogel article was obtained.

Example 2:

In Step (1), 1.5 g of aluminum hydroxide was used as the cross-linking agent, and in Step (3), cross-linking was made to proceed in an environment at 50°C. Other than the above, the same operation as in Example 1 was performed, to obtain a carbon dioxide-containing hydrogel article.

Example 3:

In Step (1), 10.0 g of tartaric acid was used instead of succinic acid, and also 50.0 g of glycerin was used. Further, 30.0 g of sodium carboxymethyl cellulose, 30.0 g of polyacrylic acid, and 20.0 g of sodium polyacrylate were used as water-soluble polymers, and no polyvinyl alcohol was included.
Other than the above, the same operation as in Example 1 was performed, to obtain a carbon dioxide-containing hydrogel article.

Example 4:

In Step (2), the carbon dioxide gas concentration in the ambient atmosphere when enclosing the hydrogel article was set to 61%. Further, in Step (3), cross-linking of the hydrogel was made to proceed until the hydrogel's loss tangent was 0.29.
Other than the above, the same operation as in Example 1 was performed, to obtain a carbon dioxide-containing hydrogel article.

Example 5:

In Step (2), the carbon dioxide gas concentration in the ambient atmosphere when enclosing the hydrogel article was set to 31%. Further, in Step (3), cross-linking of the hydrogel was made to proceed until the hydrogel's loss tangent was 0.28.
Other than the above, the same operation as in Example 1 was performed, to obtain a carbon dioxide-containing hydrogel article.

Example 6:

In Step (2), the carbon dioxide gas concentration in the ambient atmosphere when enclosing the hydrogel article was set to 15%. Further, in Step (3), cross-linking of the hydrogel was made to proceed until the hydrogel's loss tangent was 0.28.
Other than the above, the same operation as in Example 1 was performed, to obtain a carbon dioxide-containing hydrogel article.

Example 7:

In Step (1), 0.4 g of magnesium aluminometasilicate and 0.1 g of aluminum hydroxide were used as cross-linking agents. Further, in Step (2), two sheets of die-cut hydrogel articles were sealed inside a package when the loss tangent (1 Hz) of the hydrogel in the hydrogel article was 0.58. Further, in Step (3), cross-linking of the hydrogel was made to proceed until the hydrogel's loss tangent was 0.31.
Other than the above, the same operation as in Example 1 was performed, to obtain a carbon dioxide-containing hydrogel article.

Example 8:

In Step (1), 0.4 g of magnesium aluminometasilicate and 0.01 g of aluminum hydroxide were used as cross-linking agents.
Other than the above, the same operation as in Example 7 was performed, to obtain a carbon dioxide-containing hydrogel article.

Example 9:

In Step (1), 0.3 g of magnesium aluminometasilicate and 0.1 g of aluminum hydroxide were used as cross-linking agents. Further, in Step (2), two sheets of die-cut hydrogel articles were sealed inside a package when the loss tangent (1 Hz) of the hydrogel in the hydrogel article was 0.62. Further, in Step (3), cross-linking of the hydrogel was made to proceed until the hydrogel's loss tangent was 0.43.
Other than the above, the same operation as in Example 1 was performed, to obtain a carbon dioxide-containing hydrogel article.

Example 10:

In Step (2), two sheets of die-cut hydrogel articles were sealed inside a package when the loss tangent (1 Hz) of the hydrogel in the hydrogel article was 0.49. Further, in Step (3), cross-linking of the hydrogel was made to proceed until the hydrogel's loss tangent was 0.42.
Other than the above, the same operation as in Example 9 was performed, to obtain a carbon dioxide-containing hydrogel article.

Comparative Example 1:

In Step (2), the carbon dioxide gas concentration in the ambient atmosphere when enclosing the hydrogel article was set to 6%. Further, in Step (3), cross-linking of the hydrogel was made to proceed until the hydrogel's loss tangent was 0.28.
Other than the above, the same operation as in Example 1 was performed, to obtain a carbon dioxide-containing hydrogel article.

Comparative Example 2:

In Step (1), 1.5 g of aluminum hydroxide was used as a cross-linking agent. Further, carbon dioxide gas was charged after performing aging at 50°C for 2 days until the hydrogel's loss tangent was 0.26. Hence, in this Comparative Example, Step (3) was not performed. Other than the above, the same operation as in Example 1 was performed, to obtain a carbon dioxide-containing hydrogel article.

Comparative Example 3:

In Step (1), 0.5 g of magnesium aluminometasilicate was used as a cross-linking agent. Further, carbon dioxide gas was charged after performing aging at 50°C for 2 days until the hydrogel's loss tangent was 0.31. Further, the carbon dioxide gas concentration in the ambient atmosphere when enclosing the hydrogel article was set to 61%.
Other than the above, the same operation as in Comparative Example 2 was performed, to obtain a carbon dioxide-containing hydrogel article.

Comparative Example 4:

The carbon dioxide gas concentration in the ambient atmosphere when enclosing the hydrogel article was set to 31%. Other than the above, the same operation as in Comparative Example 2 was performed, to obtain a carbon dioxide-containing hydrogel article.

Comparative Example 5:

The carbon dioxide gas concentration in the ambient atmosphere when enclosing the hydrogel article was set to 15%. Other than the above, the same operation as in Comparative Example 2 was performed, to obtain a carbon dioxide-containing hydrogel article.

Comparative Example 6:

The carbon dioxide gas concentration in the ambient atmosphere when enclosing the hydrogel article was set to 6%. Other than the above, the same operation as in Comparative Example 2 was performed, to obtain a carbon dioxide-containing hydrogel article.

Evaluation:

For each of the carbon dioxide-containing hydrogel articles obtained in the Examples and Comparative Examples, the carbon dioxide gas concentration in the hydrogel was measured according to the method described below. Also, the daily production output of carbon dioxide-containing hydrogel articles was calculated for each of the Examples and Comparative Examples. Although the supply amount of the hydrogel is not particularly limited, in the present evaluation, the total gel supply amount was fixed at 3000 kg in each example. Further, for each of the carbon dioxide-containing hydrogel articles obtained in the Examples and Comparative Examples, the bubble ratio and the percentage of the total area of bubbles having virtual diameters of 5 mm or greater within the surface area of the carbon dioxide-containing hydrogel article (assumed as 100%) were measured. Furthermore, for each of the carbon dioxide-containing hydrogel articles obtained in the Examples and Comparative Examples, the adhesiveness of the article was measured and evaluated according to the following method. The results are shown in Tables 1 and 2 below.

Note that, in cases where the daily production output of carbon dioxide-containing hydrogel articles is 15000 pieces or greater, it can be assessed that the carbon dioxide-containing hydrogel articles can be produced efficiently to a sufficient degree. Also, in cases where the hydrogel's carbon dioxide gas concentration is 250 ppm or greater, it can be assessed that the carbon dioxide-containing hydrogel article is effective in promoting blood circulation.

Measurement of Carbon Dioxide Gas Concentration:

The carbon dioxide gas concentration in the carbon dioxide-containing hydrogel article was measured immediately after opening the package. More specifically, first, the carbon dioxide-containing hydrogel article was immersed in an alkaline solution placed in a lidded container, to dissolve the gel and convert the carbon dioxide gas into a carbonate. Next, the solution was made acidic again with an acidic buffer solution (pH 4.5), and the concentration of the produced carbon dioxide gas was measured with a carbon dioxide electrode (CE-2041 from DKK-Toa Corporation). From the obtained carbon dioxide gas concentration, the carbon dioxide gas concentration in the carbon dioxide-containing hydrogel article was calculated. The value of the carbon dioxide gas concentration can serve as an index for evaluating the carbon dioxide retainability of the obtained carbon dioxide-containing hydrogel article.

Evaluation of Adhesiveness of Carbon Dioxide-Containing Hydrogel Article:

After opening the package containing the carbon dioxide-containing hydrogel article and taking out the article, the article was promptly attached to the calf of an expert panelist, and the expert panelist performed dorsiflexion and plantar flexion 10 times.

Next, the area of sections that have peeled off from the calf-i.e., sections of the carbon dioxide-containing hydrogel article attached to the calf which bulge up from the calf-was measured. Then, the percentage (%) of the area of the peeled sections with respect to the entire area of the carbon dioxide-containing hydrogel article was calculated. The obtained percentage was evaluated according to the following criterion, as an index of evaluation of the adhesiveness of the carbon dioxide-containing hydrogel article. Note that, the higher the evaluation score, the better the adhesiveness. Good adhesiveness means that attachability and conformability are also good.

Evaluation Criterion of Adhesiveness:

6: The percentage of the area of the peeled sections is over 0% to 10% or less.
5: The percentage of the area of the peeled sections is over 10% to 20% or less.
4: The percentage of the area of the peeled sections is over 20% to 30% or less.
3: The percentage of the area of the peeled sections is over 30% to 40% or less.
2: The percentage of the area of the peeled sections is over 40% to 50% or less.
1: The percentage of the area of the peeled sections is over 50%.

[Table 1]

| Components | | Example 1 | Example 2 | Example 3 | Example 4 | Example 5 | Example 6 | Comparative Example 1 | Example 7 | Example 8 | Example 9 | Example 10 |
|---|---|---|---|---|---|---|---|---|---|---|---|---|
| | | Blending amount [g] | Blending amount [g] | Blending amount [g] | Blending amount [g] | Blending amount [g] | Blending amount [g] | Blending amount [g] | Blending amount [g] | Blending amount [g] | Blending amount [g] | Blending amount [g] |
| Hydrogel layer | Sodium carboxymethyl cellulose | 28.0 | 28.0 | 30.0 | 28.0 | 28.0 | 28.0 | 28.0 | 28.0 | 28.0 | 28.0 | 28.0 |
| | Polyacrylic acid | 0 | 0 | 30.0 | 0 | 0 | 0 | 0 | 0 | 0 | 0 | 0 |
| | Sodium polyacrylate | 0 | 0 | 200 | 0 | 0 | 0 | 0 | 0 | 0 | 0 | 0 |
| Tack enhancer | Polyvinyl alcohol | 50.0 | 50.0 | 0 | 50.0 | 50.0 | 50.0 | 50.0 | 50.0 | 50.0 | 50.0 | 50.0 |
| Cross-linking agent | Magnesium aluminometasilicate | 0.5 | 0 | 0 | 0.5 | 0.5 | 0.5 | 0.5 | 0.4 | 0.4 | 0.3 | 0.3 |
| | Aluminum hydroxide | 0 | 1.5 | 0.5 | 0 | 0 | 0 | 0 | 0.1 | 0.01 | 0.1 | 0.1 |
| pH adjuster | Succinic acid | 5.0 | 5.0 | 0 | 5.0 | 5.0 | 5.0 | 5.0 | 5.0 | 5.0 | 5.0 | 5.0 |
| | Tartaric acid | 0 | 0 | 100 | 0 | 0 | 0 | 0 | 0 | 0 | 0 | 0 |
| Moisturizer | Glycerin | 100.0 | 100.0 | 50.0 | 100.0 | 100.0 | 100.0 | 100.0 | 100.0 | 100.0 | 100.0 | 100.0 |
| | Propylene glycol | 50.0 | 50.0 | 50.0 | 50.0 | 50.0 | 50.0 | 50.0 | 50.0 | 50.0 | 50.0 | 50.0 |
| Other | Methyl parahydroxybenzoate | 2.5 | 2.5 | 2.5 | 2.5 | 2.5 | 2.5 | 2.5 | 2.5 | 2.5 | 2.5 | 2.5 |
| Water | Purified water | 764 | 763 | 807 | 764 | 764 | 764 | 764 | 764 | 764.09 | 764.1 | 764.1 |
| Support | | Nonwoven fabric | Nonwoven fabric | Nonwoven fabric | Nonwoven fabric | Nonwoven fabric | Nonwoven fabric | Nonwoven fabric | Nonwoven fabric | Nonwoven fabric | Nonwoven fabric | Nonwoven fabric |
| Carbon dioxide gas concentration (vol%) in ambient atmosphere when enclosing hydrogel article | | 90 | 90 | 90 | 61 | 31 | 15 | 6 | 90 | 90 | 90 | 90 |
| Loss tangent (1Hz) of hydrogel layer at Step (2) | | 0.50 | 0.61 | 0.70 | 0.50 | 0.50 | 0.50 | 0.50 | 0.58 | 0.58 | 0.62 | 0.49 |
| Loss tangent (1Hz) of hydrogel layer at Step (3) | | 0.30 | 0.21 | 0.37 | 0.29 | 0.28 | 0.28 | 0.28 | 0.31 | 0.31 | 0.43 | 0.42 |

(continued)

| | Components | Example 1 | Example 2 | Example 3 | Example 4 | Example 5 | Example 6 | Comparative Example 1 | Example 7 | Example 8 | Example 9 | Example 10 |
|---|---|---|---|---|---|---|---|---|---|---|---|---|
| | | Blending amount [g] | Blending amount [g] | Blending amount [g] | Blending amount [g] | Blending amount [g] | Blending amount [g] | Blending amount [g] | Blending amount [g] | Blending amount [g] | Blending amount [g] | Blending amount [g] |
| Evaluation | Daily production output (pieces) | 55000 | 20000 | 55000 | 55000 | 55000 | 55000 | 55000 | 55000 | 55000 | 55000 | 25000 |
| | Carbon dioxide gas concentration in article (ppm) | 1520 | 1650 | 1320 | 1109 | 539 | 281 | 171 | 1620 | 1600 | 1720 | 1480 |
| Area (%) of bubbles having virtual diameters of 5 mm or greater within hydrogel layer's surface area (assumed as 100%) | | 0.0 | 4.5 | 00 | 00 | 00 | 00 | 00 | 3.8 | 4.3 | 5.0 | 4.3 |
| Bubble ratio (%) | | 30 | 35 | 14 | 30 | 28 | 28 | 27 | 35 | 35 | 36 | 28 |
| Adhesiveness | | 5 | 4 | 5 | 5 | 5 | 5 | 5 | 5 | 5 | 6 | 6 |
| Time (hours) required until loss tangent becomes less than 0.46 | | 96 | 48 | 144 | 96 | 96 | 96 | 96 | 144 | 168 | 240 | 240 |

[Table 2]

| | Components | Comparative Example 2 Blending amount [g] | Comparative Example 3 Blending amount [g] | Comparative Example 4 Blending amount [g] | Comparative Example 5 Blending amount [g] | Comparative Example 6 Blending amount [g] |
|---|---|---|---|---|---|---|
| Hydrogel layer | Sodium carboxymethyl cellulose | 28 | 28 | 28 | 28 | 28 |
| | Polyacrylic acid | 0 | 0 | 0 | 0 | 0 |
| | Sodium polyacrylate | 0 | 0 | 0 | 0 | 0 |
| Tack enhancer | Polyvinyl alcohol | 50.0 | 50.0 | 50.0 | 50.0 | 50.0 |
| | Magnesium aluminometasilicate | 0 | 0.5 | 0.5 | 0.5 | 0.5 |
| Cross-linking agent | Aluminum hydroxide | 1.5 | 0 | 0 | 0 | 0 |
| pH adjuster | Succinic acid | 5 | 5 | 5 | 5 | 5 |
| | Tartaric acid | 0 | 0 | 0 | 0 | 0 |
| Moisturizer | Glycerin | 100 | 100 | 100 | 100 | 100 |
| | Propylene glycol | 50.0 | 50.0 | 50.0 | 50.0 | 50.0 |
| Other | Methyl parahydroxybenzoate | 2.5 | 2.5 | 2.5 | 2.5 | 2.5 |
| Water | Purified water | 763 | 764 | 764 | 764 | 764 |
| Support | | Nonwoven fabric | Nonwoven fabric | Nonwoven fabric | Nonwoven fabric | Nonwoven fabric |
| Carbon dioxide gas concentration (vol%) in ambient atmosphere when enclosing hydrogel article | | 90 | 61 | 31 | 15 | 6 |
| Loss tangent (1Hz) of hydrogel layer upon charging carbon dioxide gas | | 0.26 | 0.31 | 0.31 | 0.31 | 0.31 |
| Evaluation | Daily production output (pieces) | 5500 | 7000 | 7000 | 7000 | 7000 |
| | Carbon dioxide gas concentration in article (ppm) | 1050 | 845 | 346 | 145 | 80 |
| Area (%) of bubbles having virtual diameters of 5 mm or greater within hydrogel layer's surface area (assumed as 100%) | | 0.0 | 0.0 | 0.0 | 0.0 | 0.0 |
| Bubble ratio (%) | | 4 | 12 | 5 | 5 | 4 |
| Adhesiveness | | 5 | 5 | 5 | 5 | 5 |

The results in Tables 1 and 2 show that, by employing the present production methods, a target product can be manufactured more productively than the methods of the Comparative Examples.

The results also show that, by employing the present production methods, it is possible to obtain a target product having higher retainability of carbon dioxide gas than the Comparative Examples.

Industrial Applicability

With the present invention, it is possible to productively manufacture a hydrogel article in which the hydrogel has high carbon dioxide gas retainability.

**Claims**

1. A method for producing a carbon dioxide-containing hydrogel article, the method comprising steps (1) to (3) below:

(1) a step of preparing an article including a hydrogel that contains (A) a water-soluble polymer, (B) a cross-linking agent for the water-soluble polymer, and (C) water, a loss tangent of the hydrogel at 1 Hz in dynamic viscoelasticity measurement being 0.46 or greater;

(2) a step of, while the loss tangent, at 1 Hz in dynamic viscoelasticity measurement, of the hydrogel in the article including the hydrogel is 0.46 or greater,

adjusting composition of an ambient atmosphere of the article including the hydrogel such that a carbon dioxide gas concentration in the ambient atmosphere becomes 10 vol% or greater, and sealing the article including the hydrogel inside a package; and

(3) a step of making cross-linking of the hydrogel proceed until the loss tangent, at 1 Hz in dynamic viscoelasticity measurement, of the hydrogel in the article including the hydrogel becomes less than 0.46, and thereby obtaining a carbon dioxide-containing hydrogel article sealed inside the package.

2. The method for producing a carbon dioxide-containing hydrogel article according to claim 1, wherein the step (2) is performed at atmospheric temperature and atmospheric pressure.

3. The method for producing a carbon dioxide-containing hydrogel article according to claim 1 or 2, wherein:

the water-soluble polymer contains an anionic polymer; and the cross-linking agent contains an aluminum ion-supplying cross-linking agent.

4. The method for producing a carbon dioxide-containing hydrogel article according to any one of claims 1 to 3, wherein the step (1) comprises a step of applying the prepared hydrogel onto a support, and forming, on the support, the hydrogel article including the hydrogel.

5. The method for producing a carbon dioxide-containing hydrogel article according to any one of claims 1 to 4, wherein, in the step (1), the cross-linking agent is included such that a content of the cross-linking agent in the hydrogel is from 0.01 to 10 mass%.

Fig. 1

## INTERNATIONAL SEARCH REPORT

International application No.

PCT/JP2021/003233

A. CLASSIFICATION OF SUBJECT MATTER
Int. Cl. A61K8/02(2006.01)i, A61K8/19(2006.01)i, A61K8/26(2006.01)i,
A61K8/73(2006.01)i, A61K8/81(2006.01)i, A61K9/70(2006.01)i, A61Q19/00(2006.01)i
FI: A61K8/02, A61K8/19, A61K8/26, A61K8/73, A61K8/81, A61K9/70, A61Q19/00

According to International Patent Classification (IPC) or to both national classification and IPC

B. FIELDS SEARCHED

Minimum documentation searched (classification system followed by classification symbols)
Int. Cl. A61K8/00-8/99, A61K9/70, A61Q1/00-90/00

Documentation searched other than minimum documentation to the extent that such documents are included in the fields searched
Published examined utility model applications of Japan     1922-1996
Published unexamined utility model applications of Japan   1971-2021
Registered utility model specifications of Japan           1996-2021
Published registered utility model applications of Japan   1994-2021

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2010-202609 A (KAO CORP.) 16 September 2010, claims 1-7, paragraphs [0019], [0044]-[0048], experimental example 1 | 1-5 |
| A | JP 2006-89459 A (KAO CORP.) 06 April 2006, claims 1-5 | 1-5 |
| A | JP 2006-28158 A (KAO CORP.) 02 February 2006, claim 1, paragraph [0023] | 1-5 |
| A | JP 2005-170937 A (KAO CORP.) 30 June 2005, claims 1-9, paragraph [0015] | 1-5 |
| A | JP 2009-173649 A (KAO CORP.) 06 August 2009, claims 1-3, paragraph [0056] | 1-5 |

☒ Further documents are listed in the continuation of Box C.　　☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08.04.2021 | 20.04.2021 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

22

**INTERNATIONAL SEARCH REPORT**

International application No.

PCT/JP2021/003233

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2013-60504 A (THE UNIVERSITY OF TOKYO) 04 April 2013, claims 1-8 | 1-5 |
| A | JP 2007-269712 A (KANEBO COSMETICS INC.) 18 October 2007, claims 1-3 | 1-5 |

Form PCT/ISA/210 (continuation of second sheet) (January 2015)

International application No.

PCT/JP2021/003233

| Patent Documents referred to in the Report | Publication Date | Patent Family | Publication Date |
|---|---|---|---|
| JP 2010-202609 A | 16.09.2010 | (Family: none) | |
| JP 2006-89459 A | 06.04.2006 | (Family: none) | |
| JP 2006-28158 A | 02.02.2006 | (Family: none) | |
| JP 2005-170937 A | 30.06.2005 | (Family: none) | |
| JP 2009-173649 A | 06.08.2009 | WO 2009/084234 A1 CN 101903019 A | |
| JP 2013-60504 A | 04.04.2013 | WO 2013/039071 A1 | |
| JP 2007-269712 A | 18.10.2007 | (Family: none) | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014062087 A

- US 2020100991 A1